# EUROPEAN PATENT SPECIFICATION

(11) **EP 1 838 361 B1**
(45) Date of publication and mention of the grant of the patent: **15.07.2009**
(21) Application number: 05853391.0
(22) Date of filing: 08.12.2005
(51) Int. Cl.: A61L 27/54, A61L 29/16, A61L 31/16, A61L 27/40, A61L 29/12, A61L 31/12

(54) **MEDICAL DEVICES HAVING VAPOR DEPOSITED NANOPOROUS COATINGS FOR CONTROLLED THERAPEUTIC AGENT DELIVERY**
MEDIZINISCHE VORRICHTUNG MIT AUFGEDAMPFTEN NANOPORÖSEN BESCHICHTUNGEN ZUR GESTEUERTEN THERAPIEWIRKSTOFFABGABE
DISPOSITIFS MEDICAUX DOTES DE REVETEMENTS NANOPOREUX DEPOSES PAR EVAPORATION SOUS VIDE DESTINES A L'ADMINISTRATION REGULEE D'UN AGENT THERAPEUTIQUE

(30) Priority: 09.12.2004 US 7877
(43) Date of publication of application: 03.10.2007
(73) Proprietor: Boston Scientific Limited, Barbados, West Indies (BB)
(72) Inventor: HELMUS, Michael, N., Worcester, MA 01609 (US)
(74) Representative: Jungblut, Bernhard Jakob
(86) International application number: PCT/US2005/044461
(87) International publication number: WO 2006/063157

(56) References cited:
- WO-A-00/10622
- US-A- 5 609 629
- US-A1- 2003 130 206
- US-B1- 6 335 029
- US-B1- 6 716 444

## Description

### TECHNICAL FIELD

This invention relates to therapeutic-agent containing medical devices, and more particularly to medical devices having vapor deposited nanoporous coatings which control therapeutic agent release.

### BACKGROUND OF THE INVENTION

The in-situ presentation and/or delivery of biologically active agents within the body of a patient is common in the practice of modem medicine. In-situ presentation and/or delivery of biologically active agents are often implemented using medical devices that may be temporarily or permanently placed at a target site within the body. These medical devices can be maintained, as required, at their target sites for short or prolonged periods of time, in order to deliver biologically active agent to the target site.

Nanoporous materials have the potential to revolutionize drug delivery.

For example, iMEDD, Inc. has created silicon membranes with parallel channels ranging from 4 to 50 nm. Diffusion rates of various solutes through such membranes have been measured and conform to zero-order kinetics in some instances (i.e., release is constant with time). In general, drug diffusion rates are expected to decay with time, because the concentration gradient, and thus the driving force for diffusion, is also decaying with time. One explanation for zero order behavior is that, by making the diameter of the nanopores only slightly larger than that of the drug, the nanopores act as bottlenecks, forcing the drugs to proceed in a substantially single-file fashion through the membrane. iMedd claims that the membranes can be engineered to control rates of diffusion by adjusting channel width in relation to the size of solutes. When the proper balance is struck, zero-order diffusion kinetics is possible. iMedd has subsequently produced a drug delivery device which consists of a drug-filled enclosure which is fitted with a nanoporous membrane as the only connection between the internal reservoir of the device and the external medium.
The document WO00/10622 discloses a pores layer for controlling the release of an agent, wherein the deposited polymer has comparatively large pores. These large pores may become smaller upon further deposition.
The document US6,716,444A discloses a pores payer for controlling the release of an agent. The release rates shown exhibit a very distinct dependency from time.
The document US6,335,029 discloses a barrier layer for controlling the release of an agent. As evident from the data shown the transport kinetics is not zero-order.
The documents US2003/130206 and US5,609,629 disclose barrier layers controlling the release of an agent, wherein the barrier layer comprise pores, which are not nonopores.

### SUMMARY OF THE INVENTION

The present invention takes a different approach and is directed to medical devices according to claim 1.

In some embodiments, the lateral dimensions of the nanopores within the nanoporous coatings of the present invention are controlled such that they approach the hydrated radius of the biologically active agent.

An advantage of the present invention is that medical devices are provided, which release biologically active agents in a highly controlled fashion after administration to a patient, with release profiles being zero order release .

These and other embodiments and advantages of the present invention will become immediately apparent to those of ordinary skill in the art upon review of the Detailed Description and Claims to follow.

### BRIEF DESCRIPTION OF THE DRAWINGS

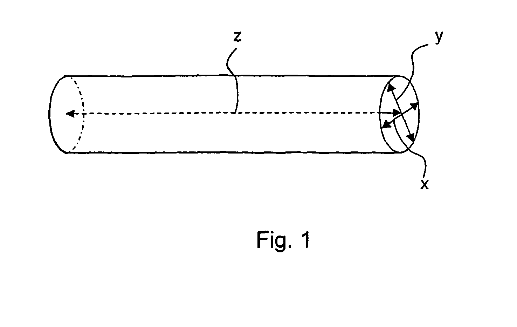

Fig. 1 is a schematic illustration of a cylindrical pore.

### DETAILED DESCRIPTION

The present invention is directed to medical devices which are adapted for controlled delivery of one or more therapeutic agents. As noted above, the medical devices of the present invention typically comprise the following: (a) an underlying region comprising the one or more therapeutic agents and (b) a vapor deposited nanoporous coating disposed over the underlying region.

"Biologically active agents," "drugs," "therapeutic agents," "pharmaceutically active agents," "pharmaceutically active materials," and other related terms may be used interchangeably herein

Vapor deposited nanoporous coatings are advantageous for a number of reasons. For example, because they are coatings, certain undesirable properties of the underlying regions, including, for example, tackiness, thrombogenicity and non-optimal vascular compatibility, among others, can be masked by the coatings.

Moreover, being vapor deposited, the nanoporous coatings of the invention are also advantageous in various embodiments, because they conform in shape to the underlying layers. Furthermore, in many embodiments, deposition techniques are employed which are not line-of-sight techniques, allowing nanoporous layers to be provided on underlying regions having highly complex three dimensional geometries.

Furthermore, because they are nanoporous, the vapor deposited coatings of the invention can be used to control release of therapeutic agents from underlying regions. For example, depending on the pore size, drug delivery devices having parallel or near parallel pore structures (e.g., the iMedd device discussed in the Background of the Invention above) can release therapeutic agent in accordance with a zero order release profile. In certain embodiments of the invention, however, medical devices are provided in which the nanoporous regions are less well defined and in which the therapeutic agent travels through the nanoporous coatings via interconnected networks of nanopores. As long as the interconnected nanopores are of sufficient size and span the thickness of the coating, therapeutic agent can migrate through the coatings. In some instances, the lateral dimensions (e.g., the radii) of the interconnected nanopores approach the lateral dimensions (e.g., the hydrated radius) of the biologically active agent that is being released. Consequently, the agent can move within, and ultimately be released from, pores of these diameters (as opposed to being trapped by pores having smaller diameters). Moreover, the interactions between the biologically active agent and the walls of the nanopores will have a significant effect upon the release profile that is observed. Indeed, as the diameter of the pore approaches the diameter of the agent to be delivered, the surface interactions begin to dominate release rates. See, e.g., Tejal A. Desai, Derek Hansford, "Mauro Ferrari Characterization of micromachined silicon membranes for immunoisolation and bioseparation applications J. Membrane Science," 159 (1999) 221-231, which describes insulin release through silicone nanomembranes. As with parallel pore structures, the systems of the present invention will release therapeutic agents in a manner that is highly controlled and they have the potential to approach zero order release kinetics. The amount of biologically active agent released and the duration of that release are also affected by the depth and tortuousity of the nanopores within the nanoporous coating.

As used herein a "nanoporous" coating is one that contains a plurality of nanopores. A "nanopore" is a void having at least one dimension that does not exceed 100 nm in length. Typically nanopores have at least two orthogonal (i.e., perpendicular) dimensions that do not exceed 100 nm and a third orthogonal dimension, which can be greater than 100 nm. By way of example, an idealized cylindrical nanopore is illustrated in Fig. 1. Being a nanopore, the cylindrical pore of Fig. 1 has at least one dimension (in this instance, the orthogonal dimensions "x" and "y") that does not exceed 100 nm in length. The third orthogonal dimension "z" of the cylindrical pore of Fig. 1 can be greater than 100 nm in length. Nanoporous coatings can further comprise pores that are not nanopores.

Nanoporous coatings in accordance with the present invention are not limited to any particular material and can be selected from a wide range of vapor deposited nanoporous metallic materials (i.e., materials formed from one or more metals), ceramic materials (i.e., materials formed from one or more ceramic materials), and polymeric materials (i.e., materials containing one or more polymers), including those listed below. Moreover, the nanoporous coatings can cover all or only a portion of the device. One or more nanoporous coating regions can be provided on the medical device surface at desired locations and/or in desired shapes (e.g., in desired patterns, for instance, using appropriate masking techniques, including lithographic techniques). For example, for tubular devices such as stents (which can comprise, for example, a laser or mechanically cut tube, one or more braided, woven, or knitted filaments, etc), nanoporous coating regions can be provided on the luminal surfaces, on the abluminal surfaces, on the lateral surfaces between the luminal and abluminal surfaces, patterned along the luminal or abluminal length of the devices, on the ends, and so forth. Moreover, multiple nanoporous coating regions can be formed using the same or different techniques, and can have the same or differing underlying biologically active agent. It is therefore possible, for example, to release the same or different therapeutic agents at different rates from different locations on the medical device. As another example, it is possible to provide a tubular tubular medical device (e.g., a vascular stent) having a first nanoporous coating disposed over a first biologically active agent (e.g., an antithrombotic agent) at its inner, luminal surface and a second nanoporous coating disposed over a second biologically active agent that differs from the first biologically active agent (e.g., an antiproliferative agent) at its outer, abluminal surface (as well as on the ends).

Examples of vapor deposition techniques coatings can be formed over underlying therapeutic-agent-containing regions in accordance with the present invention include physical and chemical vapor deposition techniques. Physical vapor deposition is typically carried out under vacuum (i.e., at pressures that are less than ambient atmospheric pressure). By providing a vacuum environment, the mean free path between collisions of vapor particles (including atoms, molecules, ions, etc.) is increased, and the concentration of gaseous contaminants is reduced, among other effects.

Physical vapor deposition (PVD) processes are processes in which a source of material, typically a solid material, is vaporized, and transported to a substrate (which, in accordance with the present invention, comprises one or more therapeutic agents) where a film (i.e., a layer) of the material is formed. PVD processes are generally used to deposit films with thicknesses in the range of a few nanometers to thousands of nanometers, although greater thicknesses are possible. PVD can take place in a wide range of gas pressures, for example, commonly within the range of 10⁻⁵ to 10⁻⁹ Torr. In many embodiments, the pressure associated with PVD techniques is sufficiently low such that little or no collisions occur between the vaporized source material and ambient gas molecules while traveling to the substrate. Hence, the trajectory of the vapor is generally a straight (line-of-sight) trajectory.

Some specific PVD methods that are used to form nanoporous coatings in accordance with the present invention include evaporation, sublimation, sputter deposition and laser ablation deposition. For instance, in some embodiments, at least one source material is evaporated or sublimed, and the resultant vapor travels from the source to a substrate, resulting in a deposited layer on the substrate. Examples of sources for these processes include resistively heated sources, heated boats and heated crucibles, among others. Sputter deposition is another PVD process, in which surface atoms or molecules are physically ejected from a surface by bombarding the surface (commonly known as a sputter target) with high-energy ions. As above, the resultant vapor travels from the source to the substrate where it is deposited. Ions for sputtering can be produced using a variety of techniques, including arc formation (e.g., diode sputtering), transverse magnetic fields (e.g., magnetron sputtering), and extraction from glow discharges (e.g., ion beam sputtering), among others. One commonly used sputter source is the planar magnetron, in which a plasma is magnetically confined close to the target surface and ions are accelerated from the plasma to the target surface. Laser ablation deposition is yet another PVD process, which is similar to sputter deposition, except that vaporized material is produced by directing laser radiation (e.g., pulsed laser radiation), rather than high-energy ions, onto a source material (typically referred to as a target). The vaporized source material is subsequently deposited on the substrate.

In general, films grown at lower temperatures (e.g., where the ratio of the temperature of the substrate, Tₛ, relative to the melting point of the deposited of the film, Tₘ, is less than 0.3) produces films that tend to be more porous than films produced at higher temperatures. See http://lpcm.esm.psu.edu/∼tjy107/research.html.

Further information regarding PVD can be found in Handbook of Nanophase and Nanostructured Materials. Vol. 1. Synthesis. Zhong Lin Wang, Yi Liu, and Ze Zhang, Editors; Kluwer Academic/Plenum Publishers, Chapter 9, "Nanostructured Films and Coating by Evaporation, Sputtering, Thermal Spraying, Electro- and Electroless Deposition".

Other aspects of the invention involve the use of chemical vapor deposition (CVD) to produce nanoporous coatings on substrates (which, in accordance with the present invention, include one or more therapeutic agents). CVD is a process whereby atoms or molecules are deposited in association with a chemical reaction (e.g., a reduction reaction, an oxidation reaction, a decomposition reaction, etc.) of vapor-phase precursor species. When the pressure is less than atmospheric pressure, CVD processes are sometimes referred to as low-pressure chemical vapor deposition (LPCVD) processes. Plasma-enhanced chemical vapor deposition (PECVD) techniques are chemical vapor deposition techniques in which a plasma is employed such that the precursor gas is at least partially ionized, thereby typically reducing the temperature that is required for chemical reaction. Unlike physical vapor deposition processes above, chemical vapor deposition processes are not necessarily line-of-site processes, allowing coatings to be formed on substrates of complex geometry.

Several examples by which nanoporous polymer films are deposited by chemical vapor deposition techniques follow. For instance, it is known to deposit nanoporous silicon dielectric films (e.g., silicon oxide films such as silicon dioxide) by PECVD using organosilicate precursor compounds such as tetraethylorthosilicate (TEOS), typically in the presence of an oxidant such as N₂O, O₂, O₃, H₂O₂, etc.. See e.g., United States Patent Application No. 2002/0142579 to Vincent et al.

As another example, it is known to deposit nanoporous silicon oxycarbide films (specifically SiOCH, also known as hydrogenated silicon oxycarbide) by PECVD oxidation of(CH3)3SiH in the presence of an oxidant (i.e., N2O). See, e.g., D. Shamiryan et al., "Comparative study of SiOCH low-k films with varied porosity interacting with etching and cleaning plasma," J. Vac. Sci. Technol. B, 20(5), Sept/Oct 2002, pp. 1923-1928.

As yet another example, in hot-filament CVD, also known as pyrolytic CVD or hot-wire CVD), a precursor gas is thermally decomposed by a source of heat such as a filament. The resulting pyrolysis products then adsorb onto a substrate maintained at a lower temperature (typically around room temperature) and react to form a film. One advantage associated with pyrolytic CVD is that the underlying substrate can be maintained at or near room temperature. As a result, films can be deposited over underlying regions that comprise a wide range of therapeutic agents, including many therapeutic agents that cannot survive other higher-temperature processes due to their thermal sensitivities.

For example, in some embodiments, a fluorocarbon polymer film is prepared by exposing a fluorocarbon monomer (e.g., hexafluoropropylene oxide, among others) to a source of heat having a temperature sufficient to pyrolyze the monomer and produce a reactive species that promotes polymerization. By maintaining the substrate region in the vicinity of the reactive species and maintaining the substrate region at a substantially lower temperature than that of the heat source, deposition and polymerization of the reactive species on the structure surface are induced. In other embodiments, fluorocarbon-organosilicon copolymer films are prepared by exposing a fluorocarbon monomer (e.g., hexafluoropropylene oxide, among others) and an organosilicon monomer (e.g., hexamethylcyclotrisiloxane or octamethylcyclotetrasiloxane, among others) to the heat source. Due to the nucleation and growth mechanisms in the HFCVD processes, nanoporous films can be made using HFCVD. For further information, see, e.g., United States Patent Application No. 2003/0138645 to Gleason et al., U.S. Patent.No. 6,156,435 to Gleason et al., and K.K.S. Lau et al., "Hot-wire chemical vapor deposition (HWCVD) of fluorocarbon and organosilicon thin films.

Reactive monomers beyond those listed above can be selected, for example, from one or more of the monomers to follow: (a) acrylic acid monomers such as acrylic acid and its salt forms (e.g., potassium acrylate and sodium acrylate); acrylic acid anhydride; acrylic acid esters including alkyl acrylates (e.g., methyl acrylate, ethyl acrylate, propyl acrylate, isopropyl acrylate, butyl acrylate, sec-butyl acrylate, isobutyl acrylate, tert-butyl acrylate, hexyl acrylate, cyclohexyl acrylate, isobomyl acrylate, 2-ethylhexyl acrylate, dodecyl acrylate and hexadecyl acrylate), arylalkyl acrylates (e.g., benzyl acrylate), alkoxyalkyl acrylates (e.g., 2-ethoxyethyl acrylate and 2-methoxyethyl acrylate), haloalkyl acrylates (e.g., 2,2,2-trifluoroethyl acrylate) and cyano-alkyl acrylates (e.g., 2-cyanoethyl acrylate); acrylic acid amides (e.g., acrylamide, N-isopropylacrylamide and N,N dimethylacrylamide); and other acrylic-acid derivatives (e.g., acrylonitrile); (b) methacrylic acid monomers such as methacrylic acid and its salts (e.g., sodium methacrylate); methacrylic acid anhydride; methacrylic acid esters (methacrylates) including alkyl methacrylates (e.g., methyl methacrylate, ethyl methacrylate, isopropyl methacrylate, butyl methacrylate, isobutyl methacrylate, t-butyl methacrylate, hexyl methacrylate, cyclohexyl methacrylate, 2-ethylhexyl methacrylate, octyl methacrylate, dodecyl methacrylate, hexadecyl methacrylate, octadecyl methacrylate, aromatic methacrylates (e.g., phenyl methacrylate and benzyl methacrylate), hydroxyalkyl methacrylates (e.g., 2-hydroxyethyl methacrylate and 2-hydroxypropyl methacrylate), aminoalkyl methacrylates (e.g., diethylminoethyl methacrylate and 2-tert-butylaminoethyl methacrylate), and additional methacrylates (e.g., isobornyl methacrylate and trimethylsilyl methacrylate; and other methacrylic-acid derivatives (e.g., methacrylonitrile); (c) vinyl aromatic monomers (i.e., those having aromatic and vinyl moieties) such as unsubstituted vinyl aromatics (e.g., styrene and 2-vinyl naphthalene); vinyl substituted aromatics (e.g., α-methyl styrene); and ring-substituted vinyl aromatics including ring-alkylated vinyl aromatics (e.g., 3-methylstyrene, 4-methylstyrene, 2,4-dimethylstyrene, 2,5-dimethylstyrene, 3,5-dimethylstyrene, 2,4,6-trimethylstyrene, and 4-tert-butylstyrene), ring-alkoxylated vinyl aromatics (e.g., 4-methoxystyrene and 4-ethoxystyrene), ring-halogenated vinyl aromatics (e.g., 2-chlorostyrene, 3-chlorostyrene, 4-chlorostyrene, 2,6-dichlorostyrene, 4-bromostyrene and 4-fluorostyrene) and ring-ester-substituted vinyl aromatics (e.g., 4-acetoxystyrene); (d) vinyl monomers (other than vinyl aromatic monomers) such as vinyl alcohol; vinyl esters (e.g., vinyl benzoate, vinyl 4-tert-butyl benzoate, vinyl cyclohexanoate, vinyl pivalate, vinyl trifluoroacetate and vinyl butyral); vinyl amines (e.g., 2-vinyl pyridine, 4-vinyl pyridine, and vinyl carbazole); vinyl halides (e.g., vinyl chloride and vinyl fluoride); alkyl vinyl ethers (e.g., methyl vinyl ether, ethyl vinyl ether, propyl vinyl ether, butyl vinyl ether, isobutyl vinyl ether, 2-ethylhexyl vinyl ether, dodecyl vinyl ether, tert-butyl vinyl ether and cyclohexyl vinyl ether); and other vinyl compounds (e.g., 1-vinyl-2-pyrrolidone and vinyl ferrocene); (e) aromatic monomers (other than vinyl aromatics) such as acenaphthalene and indene; (f) cyclic ether monomers such as tetrahydrofuran, trimethylene oxide, ethylene oxide, propylene oxide, methyl glycidyl ether, butyl glycidyl ether, allyl glycidyl ether, epibromohydrin, epichlorohydrin, 1,2-epoxybutane, 1,2-epoxyoctane and 1,2-epoxydecane; (g) ester monomers (other than previously described) such as ethylene malonate, vinyl acetate and vinyl propionate; (h) alkene monomers such as unsubstituted alkene monomers (e.g., ethylene, propylene, isobutylene, 1-butene, trans-butadiene, 4-methyl pentene, 1-octene, 1-octadecene, and other α-olefins as well as cis-isoprene and trans-isoprene) and halogenated alkene monomers (e.g., vinylidene chloride, vinylidene fluoride, cis-chlorobutadiene, trans-chlorobutadiene, and tetrafluoroethylene); and (h) organo-siloxane monomers such as dimethylsiloxane, diethylsiloxane, methylethylsiloxane, methylphenylsiloxane and diphenylsiloxane.

Using the above and other vapor deposition techniques, nanoporous coatings can be formed over a wide range therapeutic-agent-containing regions.

For instance, in some embodiments, a nanoporous coating is formed over an underlying region that comprises one or more therapeutic agents dispersed within a support material, for example, within a polymeric, ceramic or metallic support material. In other embodiments, a nanoporous coating is formed over an underlying region that includes (a) a layer that comprises one or more therapeutic-agents and, optionally, one or more additional materials (e.g., a polymeric, ceramic or metallic materials), which is disposed over (b) an underlying support material. Support materials include the metallic, ceramic and polymeric materials.

In certain beneficial embodiments, the one or more therapeutic agents are disposed within a polymeric region, for example, within a polymeric support material or within a polymeric layer that is disposed over a support material. Various polymers from which polymeric regions can be formed are listed below.

Numerous techniques are available for forming polymeric regions, including thermoplastic and solvent based techniques. For example, where the polymer (or polymers) selected to form the polymeric region have thermoplastic characteristics, a variety of standard thermoplastic processing techniques can be used to form the same, including compression molding, injection molding, blow molding, spinning, vacuum forming and calendaring, as well as extrusion into sheets, fibers, rods, tubes and other cross-sectional profiles of various lengths. Using these and other techniques, entire devices or portions thereof can be made. For example, an entire stent can be extruded using the above techniques. As another example, a coating can be provided by extruding a coating layer onto a pre-existing stent. As yet another example, a coating can be coextruded with an underlying stent body. If the therapeutic agent is stable at processing temperatures, then it can be combined with the polymer(s) prior to thermoplastic processing. If not, then is can be added to a preexisting polymer region, for example, as discussed below.

When using solvent-based techniques to provide one or more therapeutic agents within a polymeric region, the polymer(s) are first dissolved or dispersed in a solvent system and the resulting mixture is subsequently used to form the polymeric region. The solvent system that is selected will typically contain one or more solvent species. The solvent system preferably is a good solvent for the polymer(s) and, where included, for the therapeutic agent(s) as well. Preferred solvent-based techniques include, but are not limited to, solvent casting techniques, spin coating techniques, web coating techniques, solvent spraying techniques, dipping techniques, techniques involving coating via mechanical suspension including air suspension, ink jet techniques, electrostatic techniques, and combinations of these processes.

In certain embodiments, a mixture containing solvent, polymer(s) and, optionally, therapeutic agent(s), is applied to a substrate to form a polymeric region. For example, the substrate can be all or a portion of an underlying support material (e.g., a metallic implantable or insertable medical device or device portion, such as a stent) to which the polymeric region is applied. On the other hand, the substrate can also be, for example, a removable substrate, such as mold or other template, from which the polymeric region is removed after solvent elimination. In still other techniques, for example, fiber forming techniques, the polymeric region is formed without the aid of a substrate.

In certain embodiments of the invention, the therapeutic agent is dissolved or dispersed in the polymer/solvent mixture, and hence co-established with the polymeric region. In certain other embodiments, the therapeutic agent is dissolved or dispersed within a solvent, and the resulting solution contacted with a previously formed polymeric region to incorporate the therapeutic agent into the polymeric region.

As noted above, metallic, ceramic and polymeric materials are used for the formation of various components of the present invention, including, for example, vapor deposited nanoporous coatings as well as various underlying regions, including support regions and layers disposed over support regions. These metallic, ceramic and polymeric can be selected from a wide range of materials, including the following.

Metallic materials for use in conjunction with the present invention can be selected, for example, from the following: metals (e.g., silver, gold, platinum, palladium, iridium, osmium, rhodium, titanium, tungsten, and ruthenium) and metal alloys such as cobalt-chromium alloys, nickel-titanium alloys (e.g., nitinol), iron-chromium alloys (e.g., stainless steels, which contain at least 50% iron and at least 11.5% chromium), cobalt-chromium-iron alloys (e.g., elgiloy alloys), and nickel-chromium alloys (e.g., inconel alloys), among others.

Ceramic materials, including glass-ceramic and mineral materials, for use in conjunction with the present invention can be selected, for example, from the following: calcium phosphate ceramics (e.g., hydroxyapatite); calcium-phosphate glasses, sometimes referred to as glass ceramics (e.g., bioglass); various oxides, including non-transition-metal oxides (e.g., oxides of metals and semiconductors from groups 13, 14 and 15 of the periodic table, including, for example, silicon oxide, aluminum oxide) and transition metal oxides (e.g., oxides of metals from groups 3, 4, 5, 6, 7, 8, 9, 10, 11 and 12 of the periodic table, including, for example, oxides of titanium, zirconium, hafnium, tantalum, molybdenum, tungsten, rhenium, iridium, and so forth); nitrides such as metal nitrides (e.g., titanium nitride) and semiconductor nitrides (e.g., silicon nitride); carbides such as metal carbides (e.g., titanium carbide) and semiconductor carbides (e.g., silicon carbides, and silicon oxycarbides, for instance, SiOCH, also known as hydrogenated silicon oxycarbide).

Polymeric materials for use in conjunction with the present invention can be selected, for example, from the following: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydoxyalkyl celluloses; polyoxymethylene polymers and copolymers; polyimide polymers and copolymers such as polyether block imides, polyamidimides, polyesterimides, and polyetherimides; polysulfone polymers and copolymers including polyarylsulfones and polyethersulfones; polyamide polymers and copolymers including nylon 6,6, nylon 12, polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinylacetate copolymers (EVA), polyvinylidene chlorides, polyvinyl ethers such as polyvinyl methyl ethers, polystyrenes, styrene-maleic anhydride copolymers, styrene-butadiene copolymers, styrene-ethylene-butylene copolymers (e.g., a polystyrene-polyethylene/butylene-polystyrene (SEBS) copolymer, available as Kraton® G series polymers), styrene-isoprene copolymers (e.g., polystyrene-polyisoprene-polystyrene), acrylonitrile-styrene copolymers, acrylonitrile-butadiene-styrene copolymers, styrene-butadiene copolymers and styrene-isobutylene copolymers (e.g., polyisobutylene-polystyrene block copolymers such as SIBS), polyvinyl ketones, polyvinylcarbazoles, and polyvinyl esters such as polyvinyl acetates; polybenzimidazoles; ionomers; polyalkyl oxide polymers and copolymers including polyethylene oxides (PEO); glycosaminoglycans; polyesters including polyethylene terephthalates and aliphatic polyesters such as polymers and copolymers of lactide (which includes lactic acid as well as d-,1- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of polylactic acid and polycaprolactone is one specific example); polyether polymers and copolymers including polyarylethers such as polyphenylene ethers, polyether ketones, polyether ether ketones; polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), poly-4-methyl-pen-1-enes, ethylene-alphaolefin copolymers, ethylene-methyl methacrylate copolymers and ethylene-vinyl acetate copolymers; polyolefin elastomers (e.g., santoprene), ethylene propylene diene monomer (EPDM) rubbers, fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; polyurethanes; p-xylylene polymers; polyiminocarbonates; copoly(ether-esters)such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen, elastin, chitosan, gelatin, starch, glycosaminoglycans such as hyaluronic acid; as well as blends and further copolymers of the above.

Such polymers may be provided in a variety of configurations, including cyclic, linear and branched configurations. Branched configurations include star-shaped configurations (e.g., configurations in which three or more chains emanate from a single branch point), comb configurations (e.g., graft polymers having a main chain and a plurality of branching side chains), and dendritic configurations (e.g., arborescent and hyperbranched polymers). The polymers can be formed from a single monomer (i.e., they can be homopolymers), or they can be formed from multiple monomers (i.e., they can be copolymers) which commoners can be distributed, for example, randomly, in an orderly fashion (e.g., in an alternating fashion), or in blocks.

The present invention is applicable to a wide variety of medical devices including controlled drug delivery devices and other medical devices. Medical devices for use in conjunction with the various embodiments of the present invention include devices that are implanted or inserted into the body, either for procedural uses or as implants. Examples of medical devices for use in conjunction with the present invention include orthopedic prosthesis such as bone grafts, bone plates, joint prostheses, central venous catheters, vascular access ports, cannulae, metal wire ligatures, stents (including coronary vascular stents, cerebral, urethral, ureteral, biliary, tracheal, gastrointestinal and esophageal stents), stent grafts, vascular grafts, catheters (for example, renal or vascular catheters such as balloon catheters), guide wires, balloons, filters (*e.g*., vena cava filters), tissue scaffolding devices, tissue bulking devices, embolization devices including cerebral aneurysm filler coils (e.g., Guglilmi detachable coils and metal coils), heart valves, left ventricular assist hearts and pumps, and total artificial hearts.

The medical devices of the present invention may be used for systemic treatment or for localized treatment of any mammalian tissue or organ. Examples are tumors; organs and organic systems including but not limited to the heart, coronary and peripheral vascular system (referred to overall as "the vasculature"), lungs, trachea, esophagus, brain, liver, kidney, urogenital system (including, vagina, uterus, ovaries, prostate, bladder, urethra and ureters), eye, intestines, stomach, pancreas,; skeletal muscle; smooth muscle; breast; cartilage; and bone.

As used herein, "treatment" refers to the prevention of a disease or condition, the reduction or elimination of symptoms associated with a disease or condition, or the substantial or complete elimination a disease or condition. Preferred subjects (also referred to as "patients") are vertebrate subjects, more preferably mammalian subjects and more preferably human subjects.

"Biologically active agents," "drugs," "therapeutic agents," "pharmaceutically active agents," "pharmaceutically active materials," and other related terms may be used interchangeably herein and include genetic biologically active agents, non-genetic biologically active agents and cells. Biologically active agents may be used singly or in combination. Where used in combination, one biologically active agent may provide a matrix for another biologically active agent. A wide variety of biologically active agents can be employed in conjunction with the present invention. Numerous biologically active agents are described here.

Exemplary non-genetic biologically active agents for use in connection with the present invention include: (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anti-coagulants such as D-Phe-Pro-Arg chloromethyl ketone, an RGD peptide-containing compound, heparin, hirudin, antithrombin compounds, platelet receptor antagonists, anti-thrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, antimicrobial peptides such as magainins, aminoglycosides and nitrofurantoin; (m) cytotoxic agents, cytostatic agents and cell proliferation affectors; (n) vasodilating agents; (o)agents that interfere with endogenous vasoactive mechanisms, (p) inhibitors of leukocyte recruitment, such as monoclonal antibodies; (q) cytokines; (r) hormones; and (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin.

Preferred non-genetic biologically active agents include paclitaxel, sirolimus, everolimus, tacrolimus, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel and Ridogrel.

Exemplary genetic biologically active agents for use in connection with the present invention include anti-sense DNA and RNA as well as DNA coding for: (a) anti-sense RNA, (b) tRNA or rRNA to replace defective or deficient endogenous molecules, (c) angiogenic factors including growth factors such as acidic and basic fibroblast growth factors, vascular endothelial growth factor, epidermal growth factor, transforming growth factor α and β, platelet-derived endothelial growth factor, platelet-derived growth factor, tumor necrosis factor α, hepatocyte growth factor and insulin-like growth factor, (d) cell cycle inhibitors including CD inhibitors, and (e) thymidine kinase ("TK") and other agents useful for interfering with cell proliferation. Also of interest is DNA encoding for the family of bone morphogenic proteins ("BMP's"), including BMP-2, BMP-3, BMP-4, BMP-5, BMP-6 (Vgr-1), BMP-7 (OP-1), BMP-8, BMP-9, BMP-10, BMP-11, BMP-12, BMP-13, BMP-14, BMP-15, and BMP-16. Currently preferred BMP's are any of BMP-2, BMP-3, BMP-4, BMP-5, BMP-6 and BMP-7. These dimeric proteins can be provided as homodimers, heterodimers, or combinations thereof, alone or together with other molecules. Alternatively, or in addition, molecules capable of inducing an upstream or downstream effect of a BMP can be provided. Such molecules include any of the "hedgehog" proteins, or the DNA's encoding them.

Vectors for delivery of genetic therapeutic agents include viral vectors such as adenoviruses, gutted adenoviruses, adeno-associated virus, retroviruses, alpha virus (Semliki Forest, Sindbis, etc.), lentiviruses, herpes simplex virus, replication competent viruses (e.g., ONYX-015) and hybrid vectors; and non-viral vectors such as artificial chromosomes and mini-chromosomes, plasmid DNA vectors (e.g., pCOR), cationic polymers (e.g., polyethyleneimine, polyethyleneimine (PEI)), graft copolymers (e.g., polyether-PEI and polyethylene oxide-PEI), neutral polymers PVP, SP1017 (SUPRATEK), lipids such as cationic lipids, liposomes, lipoplexes, nanoparticles, or microparticles, with and without targeting sequences such as the protein transduction domain (PTD).

Cells for use in connection with the present invention include cells of human origin (autologous or allogeneic), including whole bone marrow, bone marrow derived mono-nuclear cells, progenitor cells (e.g., endothelial progenitor cells), stem cells (e.g., mesenchymal, hematopoietic, neuronal), pluripotent stem cells, fibroblasts, myoblasts, satellite cells, pericytes, cardiomyocytes, skeletal myocytes or macrophage, or from an animal, bacterial or fungal source (xenogeneic), which can be genetically engineered, if desired, to deliver proteins of interest.

Numerous biologically active agents, not necessarily exclusive of those listed above, have been identified as candidates for vascular treatment regimens, for example, as agents targeting restenosis. Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) ACE inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate, nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

Numerous additional biologically active agents useful for the practice of the present invention are also disclosed in U.S. Patent No. 5,733,925 assigned to NeoRx Corporation.

A range of biologically active agent loading levels can be used in connection with the various embodiments of the present invention, with the amount of loading being readily determined by those of ordinary skill in the art and ultimately depending, for example, upon the condition being treated, the nature of the biologically active agent, the means by which the biologically active agent is administered to the intended subject, and so forth.

## Claims

1. A medical device comprising (a) an underlying region that comprises a therapeutic agent and (b) a vapor deposited nanoporous coating over said underlying region, said vapor deposited nanoporous coating regulating the release of said therapeutic agent from said medical device when placed into a subject,
wherein said release is zero order release.

2. The medical device of claim 1,
wherein said vapor deposited nanoporous coating is a polymeric, ceramic, or metallic coating, or
wherein said nanoporous coating is deposited by physical vapor deposition, chemical vapor deposition, or
wherein said nanoporous coating is deposited by plasma enhanced chemical vapor deposition and preferably is a silicon oxide coating, in particular a silicon oxycarbide coating, e.g. a hydrogenated silicon oxycarbide coating, or
wherein said nanoporous coating is deposited by pyrolytic chemical vapor deposition and preferably comprises a fluorocarbon polymer or copolymer, a silicone polymer or copolymer, or a polymer or copolymer formed from one or more addition-polymerizable unsaturated monomers.

3. The medical device of claim 1,
wherein said underlying region comprises a plurality of different therapeutic agents, or
wherein said underlying region comprises a therapeutic agent dispersed within a support region,
wherein said support region optionally comprises a polymer, or
wherein said underlying region comprises (a) a therapeutic-agent containing coating comprising a therapeutic agent disposed over (b) an underlying support region, wherein said therapeutic-agent-containing coating optionally comprises said therapeutic agent and a polymer or consists essentially of said therapeutic agent, or wherein said underlying support region optionally is a metallic support region.

4. The medical device of claim 1, wherein the lateral dimensions of said interconnected nanopores approach the hydrated radius of said therapeutic agent.

5. The medical device of claim 1, wherein said medical device comprises a plurality of distinct nanoporous coating regions, or a plurality of distinct underlying regions.

6. The medical device of claim 1,
wherein said medical device is an implantable or insertable medical device, or
wherein said device is an implantable or insertable tubular medical device, and wherein the vapor deposited nanoporous coating is provided only on the inner luminal surface of the device, only on the outer abluminal surface of the device, or only on the edges between the luminal and abluminal surfaces of the device.

7. The medical device of claim 6,
wherein said implantable or insertable medical device is selected from catheters, guide wires, filters, stents, vascular grafts, endografts, embolic coils, heart valves, joint prostheses, bone plates and rods, dental implants, buccal implants, uterine slings, sutures, ligatures, and soft tissue reconstruction implants, or
wherein said medical device is adapted for implantation or insertion into the coronary vasculature, peripheral vascular system, esophagus, trachea, colon, biliary tract, urogenital system, or brain.

8. The medical device of claim 1, wherein said therapeutic agent is selected from one or more of the group consisting of anti-thrombotic agents, anti-proliferative agents, anti-inflammatory agents, anti-migratory agents, agents affecting extracellular matrix production and organization, anti-neoplastic agents, anti-mitotic agents, anesthetic agents, anti-coagulants, vascular cell growth promoters, vascular cell growth inhibitors, cholesterol-lowering agents, vasodilating agents, TGF-β elevating agents, and agents that interfere with endogenous vasoactive mechanisms.

9. The medical device of claim 1, wherein said device is an implantable or insertable tubular medical device that comprises a first underlying region comprising a first therapeutic agent on its inner luminal surface and a second underlying region comprising a second therapeutic agent that differs from said first biologically active agent on its outer abluminal surface, wherein said device preferably is a vascular stent and wherein said first biologically active agent is an anti-thrombotic agent and wherein said second biologically active agent is an anti-proliferative agent.

## Patentansprüche

1. Medizinische Vorrichtung mit (a) einem unterliegenden Bereich, der ein Therapeutikum aufweist, und (b) einer aufgedampfte nanoporösen Beschichtung auf dem unterliegenden Bereich, wobei die aufgedampfte nanoporöse Beschichtung die Freisetzung des Therapeutikums aus der medizinischen Vorrichtung regelt, wenn sie in ein Subjekt eingebracht ist,
wobei die Freisetzung von nullter Ordnung ist.

2. Medizinische Vorrichtung nach Anspruch 1,
wobei die aufgedampfte nanoporöse Beschichtung eine polymerische, keramische oder metallische Beschichtung ist, oder
wobei die nanoporöse Beschichtung durch physikalische Bedampfung, chemische Bedampfung aufgebracht wird, oder
wobei die nanoporöse Beschichtung durch plasmaunterstützte chemische Bedampfung aufgebracht wird und vorzugsweise eine Siliziumoxid-Beschichtung, insbesondere eine Siliziumoxycarbid-Beschichtung ist, z.B. eine hydrierte Siliziumoxycarbid-Beschichtung, oder
wobei die nanoporöse Beschichtung durch pyrolytische chemische Bedampfung aufgebracht wird und vorzugsweise ein Fluorkarbon-Polymer oder -Copolymer, ein Silizium-Polymer oder -Copolymer oder ein aus einem oder mehreren additions-polymerisierbaren ungesättigten Monomeren gebildetes Polymer oder Copolymer aufweist.

3. Medizinische Vorrichtung nach Anspruch 1,
wobei der unterliegende Bereich eine Vielzahl von verschiedenen Therapeutika aufweist, oder
wobei der unterliegende Bereich ein in einem Trägerbereich dispergiertes Therapeutikum aufweist, wobei der Trägerbereich optional ein Polymer aufweist, oder
wobei der unterliegende Bereich (a) eine ein Therapeutikum enthaltende Beschichtung mit einem auf (b) einem unterliegenden Trägerbereich angeordnetes Therapeutikum aufweist, wobei die ein Therapeutikum enthaltende Beschichtung optional das Therapeutikum und ein Polymer aufweist oder im wesentlichen aus dem Therapeutikum besteht, oder wobei der unterliegende Trägerbereich optional ein metallischer Trägerbereich ist.

4. Medizinische Vorrichtung nach Anspruch 1, wobei die seitlichen Abmessungen der verbundenen Nanoporen dem hydrierten Radius des Therapeutikums nahe kommen.

5. Medizinische Vorrichtung nach Anspruch 1, wobei die medizinische Vorrichtung eine Vielzahl von eigenständigen nanoporösen Beschichtungsbereichen oder eine Vielzahl von eigenständigen unterliegenden Bereichen aufweist.

6. Medizinische Vorrichtung nach Anspruch 1,
wobei die medizinische vorrichtung eine implantierbare oder einführbare medizinische Vorrichtung ist, oder
wobei die Vorrichtung eine implantierbare oder einführbare rohrförmige Medizinische Vorrichtung ist, und wobei die aufgedampfte nanoporöse Beschichtung nur auf der inneren luminalen Fläche der Vorrichtung, nur auf der äußeren abluminalen Fläche der Vorrichtung oder nur auf den Kanten zwischen den luminalen und abluminalen Flächen der Vorrichtung vorgesehen ist.

7. Medizinische Vorrichtung nach Anspruch 6,
wobei die implantierbare oder einführbare medizinische Vorrichtung ausgewählt ist aus Kathetern, Führungsdrähten, Filtern, Stents, Vaskulär-Grafts, Endografts, Emboliespulen, Herzklappen, Gelenkprothesen, Knochenplatten und -stangen, Zahnimplantaten, Mundimplantaten, Uterusschlingen, Nähten, Bandagen und Implantaten zum Wiederaufbau von Weichteilen, oder
wobei die medizinische Vorrichtung zum Implantieren oder Einführen in die koronare vaskulatur, das periphere vaskuläre System, die Speiseröhre, die Luftröhre, den Dickdarm, den Gallengang, das urogenitale System oder das Gehirn ausgebildet ist.

8. Medizinische Vorrichtung nach Anspruch 1, wobei das Therapeutikum ausgewählt ist aus einem oder mehreren der Gruppe bestehend aus Anti-Thrombose-Mitteln, antiproliferativen Mitteln, entzündungshemmenden Mitteln, antimigratorischen Mitteln, die extrazelluläre Matrixproduktion und -organisation beeinflussenden Mitteln, antineoplastischen Mitteln, antimitotischen Mitteln, anästhetischen Mitteln, Antikoagulantien, Gefäßzellen-Wachstumspromotern, Gefäßzellen-Wachstumsinhibitoren, cholesterinsenkenden Mitteln, gefäßerweiternden Mitteln, TGF-β erhöhenden Mitteln und Mitteln, die die endogenen vasoaktiven Mechanismen stören.

9. Medizinische Vorrichtung nach Anspruch 1, wobei die Vorrichtung eine implantierbare oder einführbare rohrförmige medizinische Vorrichtung ist, die einen ersten unterliegenden Bereich mit einem ersten Therapeutikum auf seiner inneren luminalen Fläche und einen zweiten unterliegenden Bereich mit einem zweiten Therapeutikum, das vom ersten biologisch aktiven Mittel abweicht, auf seiner äußeren abluminalen Fläche aufweist,
wobei die Vorrichtung vorzugsweise ein Vaskulär-Stent ist, und wobei das erste biologisch aktive Mittel eine Anti-Thrombose-Mittel ist, und wobei das zweite biologisch aktive Mittel ein antiproliferatives Mittel ist.

## Revendications

1. Dispositif médical comprenant (a) une région sous-jacente qui comprend un agent thérapeutique et (b) une couche nanoporeuse déposée en phase vapeur sur cette région sous-jacente, cette couche nanoporeuse déposée en phase vapeur régulant la libération de cet agent thérapeutique à partir de ce dispositif medical si mis en place dans un sujet,
dans lequel cette libération est d'ordre zéro.

2. Dispositif médical selon la revendication 1,
dans lequel cette couche nanoporeuse déposée en phase vapeur est une couche polymérique, céramique ou métallique, ou
dans lequel cette couche nanoporeuse est déposée par dépôt physique en phase vapeur, dépôt chimique en phase vapeur, ou
dans lequel cette couche nanoporeuse est déposée par dépôt chimique en phase vapeur assisté par plasma et de préférence est une couche d'oxyde de silicium, en particulier une couche d'oxycarbure de silicium, p.ex. une couche d'oxycarbure de silicium hydrogéné, ou
dans lequel cette couche nanoporeuse est déposée par dépôt chimique pyrolytique en phase vapeur et de préférence comprend un polymère ou copolymère de fluorocarbure, un polymère ou copolymère de silicium ou un polymère ou copolymère formé à partir d'un ou plusieurs monomères insaturés polymérisables par addition.

3. Dispositif médical selon la revendication 1,
dans lequel cette région sous-jacente comprend une pluralité d'agents thérapeutiques différents, ou
dans lequel cette région sous-jacente comprend un agent thérapeutique répandu dans une région de support, dans lequel cette région de support optionnellement comprend un polymère, ou
dans lequel cette région sous-jacente comprend (a) une couche contenant un agent thérapeutique comprenant un agent thérapeutique disposé sur (b) une région de support sous-jacente, dans lequel cette couche contenant un agent thérapeutique optionnellement comprend cet agent thérapeutique et un polymère ou consiste essentiellement en cet agent thérapeutique, ou dans lequel cette région de support sous-jacente optionnellement est une région de support métallique.

4. Dispositif médical selon la revendication 1, dans lequel les dimensions latérales de ces nanopores interconnectées s'approchent au rayon hydraté de cet agent thérapeutique.

5. Dispositif médical selon la revendication 1, dans lequel ce dispositif médical comprend une pluralité de régions de couches nanoporeuses distinctes, ou une pluralité de régions sous-jacentes distinctes.

6. Dispositif médical selon la revendication 1,
dans lequel ce dispositif médical est un dispositif médical implantable ou insérable, ou dans lequel ce dispositif est un dispositif médical tubulaire implantable ou insérable, et dans lequel la couche nanoporeuse déposée en phase vapeur est prévue seulement sur la surface luminale intérieure du dispositif, seulement sur la surface abluminale extérieure du dispositif ou seulement sur les arêtes entre les surfaces luminales et abluminales du dispositif.

7. Dispositif médical selon la revendication 6, dans lequel ce dispositif médical implantable ou insérable est choisi à partir de cathéters, fils de guidage, filtres, stents, greffes vasculaires, endogreffes, bobines emboliques, valves du coeur, prothèses des articulations, lames et tiges osseuses, implants dentaires, implants buccaux, bandelettes utérines, sutures, ligatures et implants pour la reconstruction des tissus mous, ou
dans lequel ce dispositif médical est agencé pour l'implantation ou insertion dans la vasculature coronaire, le système vasculaire périphérique, l'oesophage, la trachée, le côlon, les voies biliaires, le système urogénital ou le cerveau.

8. Dispositif médical selon la revendication 1,
dans lequel cet agent thérapeutique est choisi à partir d'un ou plusieurs du groupe comprenant des agents antithrombotiques, agents antiprolifératifs, agents anti-inflammatoires, agents antimigratoires, agents influençant la production et l'organisation de la matrice extracellulaire, agents antinéoplastiques, agents antimitotiques, agents anesthétiques, anticoagulants, promoteurs de la croissance des cellules vasculaires, inhibiteurs de la croissance des cellules vasculaires, agents réduisant le cholestérol, agents vasodilatateurs, agents augmentant le TGF-β et agents, qui interfèrent avec les mécanismes vasoactifs endogènes.

9. Dispositif médical selon la revendication 1,
dans lequel ce dispositif est un dispositif médical tubulaire implantable ou insérable, qui comprend une première région sous-jacente comprenant un premier agent thérapeutique sur sa surface luminale intérieure et une deuxième région sous-jacente comprenant un deuxième agent thérapeutique qui diffère de ce premier agent biologiquement actif sur sa surface abluminale extérieure, dans lequel ce dispositif est de préférence un stent vasculaire et dans lequel ce premier agent biologiquement actif est un agent antithrombotique et dans lequel ce deuxième agent biologiquement actif est un agent antiprolifératif.
